# EUROPEAN PATENT SPECIFICATION

(11) **EP 4 121 013 B1**
(45) Date of publication and mention of the grant of the patent: **15.01.2025**
(21) Application number: 21711281.2
(22) Date of filing: 16.03.2021
(51) Int. Cl.: A61K 9/20, A61K 31/137

(54) **SCORED TABLET COMPRISING TAPENTADOL**
GEKERBTE TABLETTE ENTHALTEND TAPENTADOL
COMPRIMÉ SÉCABLE COMPRENANT TAPENTADOL

(30) Priority: 16.03.2020 EP 20163336
(43) Date of publication of application: 25.01.2023
(73) Proprietor: Grünenthal GmbH, 52078 Aachen (DE)
(72) Inventor: ELLERMANN, Angelika, 64285 Darmstadt (DE); BERTRAM, Ulrike, 52066 Aachen (DE); BARTZ, Gerd, 52379 Langerwehe (DE); RÜTTGERS, Udo, 52222 Stolberg (DE)
(74) Representative: Kutzenberger Wolff & Partner
(86) International application number: PCT/EP2021/056618
(87) International publication number: WO 2021/185804

(56) References cited:
- EP-A1- 2 103 302
- WO-A1-2012/028319
- WO-A1-2014/108514
- DE-A1- 10 152 469
- GB-A- 2 057 878
- US-A1- 2011 033 537
- US-A1- 2016 199 305
- T.M. TSCHENTKE ET AL: "Tapentadol Hydrochloride", DRUGS OF THE FUTURE, vol. 31, no. 12, 1 January 2006 (2006-01-01), ES, pages 1053 - 1061, XP055365206, ISSN: 0377-8282, DOI: 10.1358/dof.2006.031.12.1047744

## Description

Priority is claimed of European patent application no. 20 163 336.9, filed on March 16, 2020.

The invention relates to a scored tablet according to claim 1. The invention further relates to a process for the preparation of such a scored tablet according to claim 9.

Many pharmaceutical dosage forms are provided as scored tablets that can be manually broken by the patient along a site of mechanical weakness such as a breaking score thereby providing two halves each containing essentially 50% of the dose of the pharmacologically active ingredient. The breaking of such pharmaceutical dosage forms can serve different purposes. In certain instances it can be desirable to begin the treatment with a reduced dose of the pharmacologically active ingredient and to subsequently increase the dose, i.e. to titrate the pharmacologically active ingredient during a titration phase, followed by a treatment phase where the administered dose is kept constant. In other instances it can be desirable for the ease of swallowing to break the dosage form apart and to take the fragments all together, i.e. within a couple of minutes by separate swallowing.

Many pharmaceutical dosage forms provide prolonged release of the pharmacologically active ingredient contained therein. In contrast to pharmaceutical dosage forms providing immediate release, such dosage forms are designed to retard the release. Retardation of release may be achieved by a film coating with an insoluble polymer having pores of defined number and size through which the pharmacologically active ingredient may by release into the environment. Alternatively, retardation of release may be achieved by a prolonged release matrix in which the pharmacologically active ingredient is embedded.

When scored tablets are broken apart, the surface at the site of mechanical weakness becomes exposed. Thus, the total surface of the two halves is greater than the surface of the intact scored tablet.

On the one hand, when the tablet is provided with a prolonged release coating, the exposed surface at the site of mechanical weakness is uncovered and provides a significantly faster release of the pharmacologically active ingredient than the other surfaces of the two halves which are covered with the prolonged release coating. In consequence, the separate halves provide an *in vitro* release profile that significantly deviates from the *in vitro* release profile of the intact scored tablet. Therefore, prolonged release coatings are typically not suitable for scored tablets.

On the other hand, when the tablet is provided with a prolonged release matrix in which the pharmacologically active ingredient is embedded, release through the exposed surface at the site of mechanical weakness typically essentially corresponds to release through the other surfaces of the two halves that are also exposed to the environment when the divisible table is intact. Nonetheless, as the total surface area is increased by breaking the tablet apart, the two halves typically show a faster release than the intact scored tablet. Thus, it makes a difference with respect to the pharmacokinetic properties whether the patent takes an intact scored tablet or whether the patient takes the two halves that were broken apart.

W.R. Primrose et al., Alteration of pharmacokinetics after halving a slow-release theophylline tablet, Pharmatherapeutica, 1983, 3(6), 429-32 report that absorption was faster and plasma levels were higher when a 400 mg Theophylline tablet formulation was taken as two separate halves.

V.P. Shah et al., Analysis of in vitro Dissolution of Whole vs. Half Controlled-Release Theophylline Tablets, Pharmaceutical Research, Vol. 4, No. 5, 1987 report about a study on different kinds of Theophylline controlled release tablets of 6 different brands with different dissolution rates. The dissolution of halved tablets was significantly faster compared to that of intact (whole) tablets.

T.K. Mandal, Effect of tablet integrity on the dissolution rate of sustained-release preparations. J Clin Pharm Ther. 1996;21(3):155-157. doi:10.1023/A:1016442514205, describes ZOR-prin sustained release tablets with respect to splitting. The split tablets showed a consistently higher release profile over time, with a 50% higher release at 6 h.

J. Erramouspe et al., Effect on dissolution from halving methylphenidate extended-release tablets. Ann Pharmacother. 1997;31(10):1123-1126. doi:10.1177/106002809703101001, relates to Methylphenidate extended release tablets presumably based on a lipophilic matrix (Cetylstearylalcohol as described in Ritalin SR Label of US product). Halving the tablets caused a statistically significant increase in cumulative dissolution as early as 15 minutes. The difference in cumulative dissolution reached its maximum for both Ritalin-SR and generic methylphenidate extended-release tablets at 2 hours. At this time point, the percent dissolution of the whole versus halved tablets was 57% versus 74% (Ritalin-SR), respectively, and 49% versus 67% (generic), respectively.

A. Sood et al., Drug release evaluation of diltiazem CR preparations. Int J Pharm. 1998;175(1):95-107. doi:10.1016/S0378-5173(98)00268-3 evaluate (a) the effects of dissolution medium pH and dosage form structural integrity on the release mechanisms and kinetics of Diltiazem HCl (DLTZ) from peroral CR/SR preparations, and (b) the CR/SR products comparatively for their drug release parameters. The split tablets showed a consistently higher release profile over time. Splitting of the tablets not only gave faster drug release, unlike the intended slower release profiles, but also increased the variability thereby reducing the reproducibility.

E. Vranic et al., Influence of Splitting on Dissolution Properties of Metoprolol Tablets. Bosn J BASIC Med Sci. 2009;9(3):245-249 compares several release profiles of dissolution data for Metropolol controlled release tablet formulations in order to identify possible changes in dissolution profiles of whole and scored tablets. The halved tablets release faster than the intact tablets.

N. Zhao et al., Tablet splitting: Product quality Assessment of metoprolol succinate extended release tablets. Int J Pharm. 2010;401(1-2):25-31. doi:10.1016/j.ijpharm. 2010.09.004 discloses that Multiple Unit Particulate System (MUPS) containing sustained release pellets compressed into a fast disintegrating matrix provide similar release of halved and intact tablets because in-vitro release is determined by the coated pellets that stay intact when splitting the MUPS tablets.

Similarly, according to M.T. Teixeira et al., The Influence of Matrix Technology on the Subdivision of Sustained Release Matrix Tablets. AAPS PharmSciTech. 2019 Dec 3;21(1):8. doi: 10.1208/s12249-019-1554-1, splitting of matrix sustained release matrix tablets is not recommended because of faster release of the split tablets; only sustained release system recommended for splitting is a Multiple Unit Particulate System (MUPS) that contains sustained release microparticles compressed into an immediate disintegrating matrix.

Summarizing therefore, splitting of sustained release matrix tablets is generally not recommended because of faster release of the split tablets.

Scored tablets can be advantageously used in dose titration, i.e. when at the beginning of therapy, the dose of the pharmacologically active ingredient is steadily to be increased until the desired therapeutically effective dose is reached. Titration can have several advantages, especially with respect to the reduction of treatment emergent adverse events (undesired side effects).

For example, a scored tablet containing a dose of 100 mg of a pharmacologically active ingredient may be split into two haves each containing 50 mg of a pharmacologically active ingredient. The individual haves may then be used for dose titration. For example on a first day of therapy, the first half of the split tablet may be administered and on the second day of therapy, the second half of the split tablet may be administered. If the dose is to be increased to 100 mg on the third day of therapy, this may then be achieved by administering the intact (whole, unsplit) tablet. For example, according to the prescribing information for Nucynta^{®} ER (Tapentadol HCl extended release), the tablets should be individually titrated to a dose that provides adequate analgesia and minimizes adverse reactions. Patients should be titrated to adequate analgesia with dose increases of 50 mg no more than twice daily every three days.

It is known that during dose titration of various pharmacologically active ingredients a significant number of patients, sometimes up to 20% or more, decide to terminate treatment because of side effects that cannot be tolerated. The incidence of side effects is typically a function of the administered dose of the pharmacologically active ingredient, whereas titration may serve the purpose of slowly adapting the patient's organism to the pharmacologically active ingredient. Thus, the organism shall become used and accustomed to the pharmacologically active ingredient, as this may significantly reduce side effects. When the onset of the plasma level of the pharmacologically active ingredient is too rapid, however, the customization process does not have sufficient time resulting in an increased incidence of side effects. Thus, when scored tablets are to be used in dose titration, it is desirable that the physiological effects (therapeutic effects and possible side effects) are controlled also when only halves of the split tablet are administered. In particular, it is desirable that under physiological conditions release kinetics of the pharmacologically active ingredient from one half (i.e. 50% of the original dose contained in the whole scored tablet before it was split) proportionally correspond to release kinetics of the pharmacologically active ingredient from the whole tablet (i.e. 100% of the dose contained in the whole scored tablet).

Further, as some patients have problems in swallowing large tablets, it is possible that these patients even during the dose maintenance phase, i.e. after dose titration, will not take the split tablet as a whole, but will split it into two halves and for the ease of swallowing will take the two halves simultaneously or shortly after one another in order to administer the desired full dose of the pharmacologically active ingredient.

There is therefore a demand for pharmaceutical dosage forms that can be divided into two parts and that provide essentially the same *in vitro* release profile irrespective of whether the intact dosage form is taken or whether the two separate halves are taken.

According to *Food and Drug Administration, Guidance for Industry* on *Tablet Scoring: Nomenclature, Labeling, and Data for Evaluation,* the split tablet should *inter alia* be safe to handle and not pose risk of unintended drug exposure. Modified release products for which the control of drug release can be compromised by tablet splitting should not have a scoring feature. The split tablet portions should meet the same finished-product testing requirements as for a whole-tablet product with equivalent strength. A risk assessment should be provided to justify the tests and criteria for product with the proposed functional scoring. The assessment should be undertaken on both tablets that are split nonme-chanically (by hand) and tablets that are split mechanically (with a tablet splitter). Dissolution on whole versus split tablet portions should meet the similarity factor (f2) criteria according to *Guidance for Industry* on *Dissolution Testing of Immediate Release Solid Oral Dosage Forms* (f₂ = 50·log{[1+(1/n)Σₜ₌₁ⁿ (Rₜ - Tₜ)²]^{-0.5}·100)). For curves to be considered similar, f₂ values should be close to 100. Generally, f₂ values greater than 50 (50-100) ensure sameness or equivalence of the two curves and, thus, of the performance of the test (postchange) and reference (prechange) products.

Further, many initially approved pharmaceutical dosage forms are no scored tablets. Thus, the initially approved dosage forms are conventional tablets, whereas it may subsequently become desirable to also provide the same pharmacologically active ingredient in form of scored tablets.

When after approval of a pharmaceutical dosage form changes are made, however, various regulatory requirements need to be satisfied. According to the European *Guideline on the details of the various categories of variations to the terms of marketing authorisations for medicinal products for human use and veterinary medicinal products* according to *Commission Regulation (EC) No. 1234*/*2008 of November 2008 concerning the examination of variations to the terms of marketing authorisations for medicinal products for human use and veterinary medicinal products,* changes in the shape or dimensions of the pharmaceutical form are included in category B.II.a.2, where scored tablets intended to be divided into equal doses are mentioned under b). Documentation to be supplied with regard to such changes inter alia include *comparative dissolution data on at least one pilot batch of the current and proposed dimensions (no significant differences regarding comparability see the relevant (Human or Veterinary) guidance on Bioavailability);* and *justification for not submitting a new bioequivalence study according to the relevant (Human or Veterinary) guidance on Bioavailability.*

Typically, the *in vitro* dissolution profile of a changed dosage form needs to be equivalent to the *in vitro* dissolution profile of the initially authorized dosage form. Therefore, when the initially authorized dosage form was a tablet not equipped with scoring properties and when the newly developed changed dosage form is a scored tablet, the *in vitro* dissolution profile of the newly developed changed dosage form must exhibit an *in vitro* dissolution profile having an f₂ value a generally greater than 50.

In-vitro in-vivo correlation (IVIVC) is a predictive mathematical model describing the relationship between an in-vitro property of a dosage form and an in-vivo response. IVIVC establishes a relationship between a biological property, or a parameter derived from a biological property produced from a dosage form, and a physicochemical property of the same dosage form. When an IVIVC model can be established, it may not be necessary to perform additional studies *in vivo* demonstration bioequivalence, bioavailability, and the like.

Post-approval changes in the composition of an approved pharmaceutical dosage form are typically regarded as more substantial than mere changes in the shape or dimensions of the pharmaceutical form. According to the European *Guideline on the details of the various categories of variations to the terms of marketing authorisations for medicinal products for human use and veterinary medicinal products* according to *Commission Regulation (EC) No. 1234*/*2008 of November 2008 concerning the examination of variations to the terms of marketing authorisations for medicinal products for human use and veterinary medicinal products,* changes in the shape or dimensions of the pharmaceutical form are included in category B.II.a.3. Changes in the composition, i.e. different excipients or different amounts thereof, may thus require not only stability tests but additionally also bioequivalence studies where pharmacokinetic parameters are determined *in vivo* such as AUC, tₘₐₓ, Cₘₐₓ, and the like. According to EMA *Note for Guidance on the Investigation of Bioavailability and Bioequivalence,* a bioequivalence study is required, unless otherwise justified, if a product has been reformulated from the formulation initially approved or the manufacturing method has been modified by the manufacturer in ways that could be considered to impact on the bioavailability.

Thus, there is also a demand for means suitable for transforming an initially approved pharmaceutical dosage form into a scored tablet without significantly changing the formulation in ways that could be considered to impact on the bioavailability. In other words, there is a demand to retain the excipients and quantities of the initially approved pharmaceutical dosage form when providing a scored tablet, as this may avoid the requirement for bioequivalence studies.

WO 03/035053 and DE 101 52 469 relate to a pharmaceutical formulation which is characterized by delayed release of the active ingredient. Said formulation contains Tapentadol or one of its pharmaceutically acceptable salts in a matrix with delayed release of the active ingredient. Said matrix contains between 1 and 80 wt. % of at least one hydrophilic or hydrophobic polymer as a pharmaceutically acceptable matrix forming agent and exhibits, *in vitro,* the following dissolution speed: between 3 and 35 wt. % (in relation to 100 wt. % of active ingredient) of Tapentadol is released after half an hour, between 5 and 50 wt. % of Tapentadol is released after one hour, between 10 and 75 wt. % of Tapentadol is released after two hours, between 15 and 82 wt. % of Tapentadol is released after three hours, between 30 and 97 wt. % of Tapentadol is released after six hours, more than 50 wt. % of Tapentadol is released after twelve hours, more than 70 wt. % of Tapentadol is released after eighteen hours, and more than 80 wt. % of Tapentadol is released after twenty-four hours.

WO 2008/128740 relates to the use of tapentadol for the manufacture of a medicament comprising at least one administration unit A containing dose a of tapentadol and at least one administration unit B containing dose b of tapentadol, where dose a < dose b, for the treatment of pain. Disclosed is a pharmaceutical oral dosage form containing tapentadol, which comprises a score, that divides the dosage form into at least two portions and mechanically weakens the dosage form so that it may be manually broken at the score along a predetermined site of fracture, which contains 40 to 260 mg of tapentadol so that, after being broken at the score along the predetermined site of fracture, each portion contains about 20 to about 130 mg of tapentadol.

WO 2009/092601 and US 2016/0199305 relate to a pharmaceutical dosage form, preferably with controlled release of a pharmacologically active compound (A) contained therein, the pharmaceutical dosage form very preferably being tamper-resistant and most preferably having a breaking strength B1 of at least 500 N in direction of extension E1 and having a breaking strength B2 of less than 500 N in direction of extension E2.

WO 2014/108514 relates to Tapentadol maleate, its polymorphic forms as well as processes for its preparation, pharmaceutical compositions, and preparation thereof.

US 2011/0033537 relates to a sustained release formulation for delivering one or more pharmaceutically active agents. The formulation comprises cross-linked high amylose starch and at least one pharmaceutically active agent, and optionally can be subdivided into smaller dosage forms where the smaller dosage forms have substantially the same sustained release properties as the formulation from which they were derived. The formulations can provide sustained release for up to at least 24 hours, and because of their divisability permits a recipient of the active agent or the person administering the active agent to titrate the dosage of the agent.

EP 2 103 302 relates to a modified release divisible tablet that comprises an active ingredient, cellulose derivative, and a binder comprising non-subdivided tablet and a fraction of the tablet obtained by a subdivision, which has an identical dissolution profile. Preparation of the tablet comprises wet granulation, where the granulation is carried out by direct compaction or compression.

GB 2 057 878 relates to a divisible tablet having controlled and delayed release of the active substance. The reference aims at providing a divisible retard tablet form that is readily divided into fragments which, between them, have an approximately equal content of active substance and active substance release characteristics approximating those obtained for the whole tablet; that is to say, it was necessary to find a tablet form which, on being divided, would produce the smallest possible increase, resulting from the formation of fracture surfaces, in the total surface area.

It is an object of the invention to provide pharmaceutical dosage forms comprising Tapentadol or a physiologically acceptable salt thereof and having advantages compared to the prior art. The pharmaceutical dosage forms should have excellent patient compliance and should provide improved safety. The composition of the pharmaceutical dosage forms should not be significantly changed in ways that could be considered to impact on the bioavailability compared to an initially approved pharmaceutical dosage form containing the same dose of Tapentadol or a physiologically acceptable salt thereof.

This object has been achieved by the subject-matter of the patent claims.

It has been surprisingly found that scored tablets can be prepared by dry granulation, especially under specific conditions of dry granulation, which can be divided into two parts and which provide essentially the same *in vitro* release profile irrespective of whether the intact dosage form is taken or whether a single separate half is taken or whether the two separate halves are taken together.

In this regard, the *in vitro* release profile is expressed in terms of the released percentage of Tapentadol or the physiologically acceptable salt thereof that was originally contained in the sample subjected to dissolution testing (whole intact tablet, or separate single half alone, or separated halves together). When considering absolute dosages of Tapentadol or the physiologically acceptable salt thereof e.g. in mg, however, it makes of course a difference whether the whole intact tablet is subjected to dissolution testing or whether a separate single half alone is subjected to dissolution testing. In the latter case, it has been surprisingly found that a separate single half alone releases at any point in time during dissolution testing essentially about 50% of the absolute amount of Tapentadol or the physiologically acceptable salt thereof (e.g. in mg) compared to the intact dosage form when tested as a whole.

A first aspect of the invention relates to a scored tablet comprising Tapentadol or a physiologically acceptable salt thereof embedded in a prolonged release matrix material; wherein
the prolonged release matrix material comprises a binder selected from microcrystalline cellulose; wherein the weight content of the binder is within the range of from 35 to 70 wt.-%, relative to the total weight of the scored tablet;
wherein the prolonged release matrix material comprises a cellulose derivative selected from hydroxypropylmethylcellulose; wherein the weight content of the cellulose derivative is within the range of from 10 to 50 wt.-%, relative to the total weight of the scored tablet;
wherein the scored tablet has been prepared by dry granulation;
wherein the scored tablet provides prolonged release of Tapentadol or the physiologically acceptable salt thereof;
wherein the scored tablet has a site of mechanical weakness along which it can be manually broken into two separate halves; and
wherein the *in vitro* release profile of the scored tablet essentially corresponds to the *in vitro* release profile of each of the two separate halves.

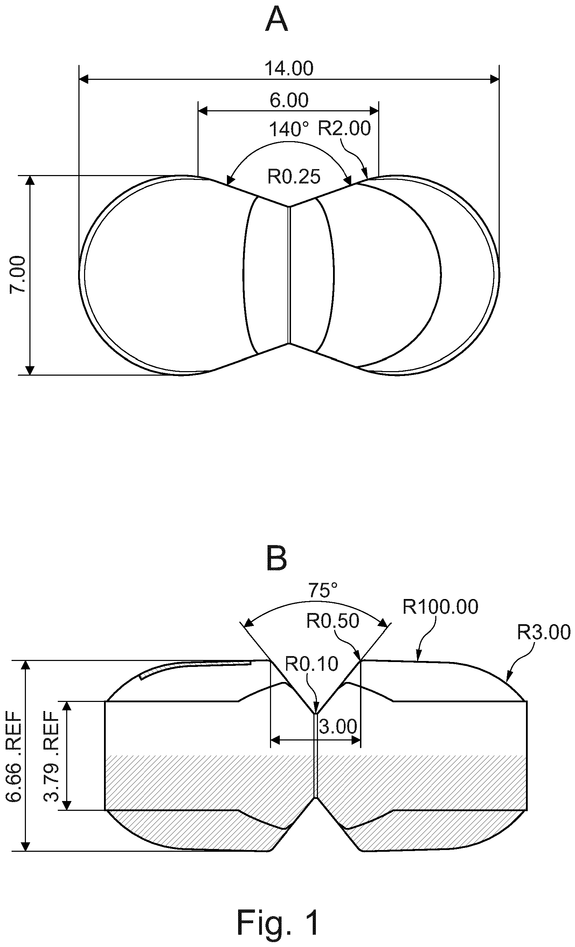

Figure 1 schematically illustrates an exemplified preferred shape and form of a scored tablet according to the invention that was also prepared in the examples. Figure 1A is a top view, Figure 1B is a side view. The scored tablet contains scores on opposite sides that form a site of mechanical weakness along which the divisible table can be broken apart into two separate halves of essentially the same form, shape and weight. The site of mechanical weakness (breaking score) lies within a symmetrical plane of the scored tablet. Figures 1A/B also provide preferred dimensions in mm defining the theoretical total volume of a preferred scored tablet. A preferred exemplified scored tablet according to the invention having this form and shape has a total weight of about 540±25 mg.

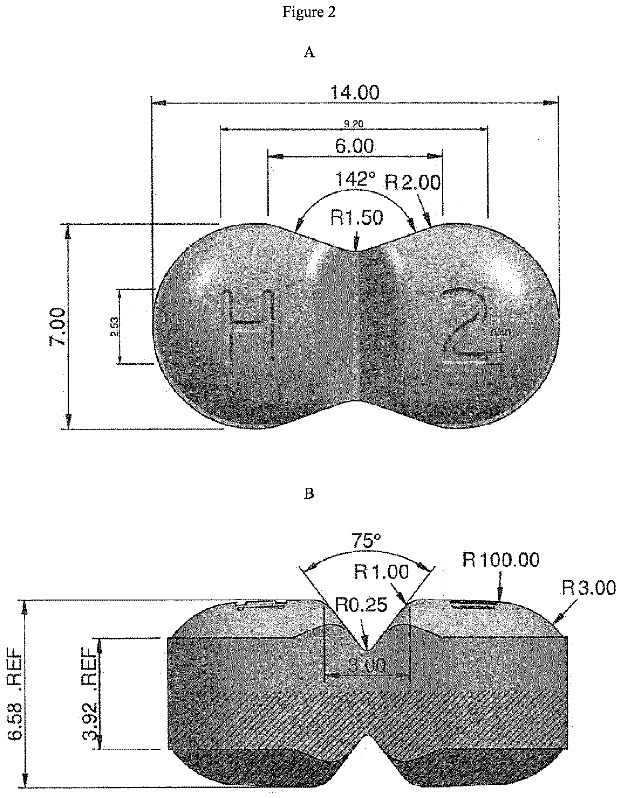

Figure 2 schematically illustrates a variant of an exemplified preferred shape and form of a scored tablet according to the invention that was also prepared in the examples. Figure 2A is a top view, Figure 2B is a side view.

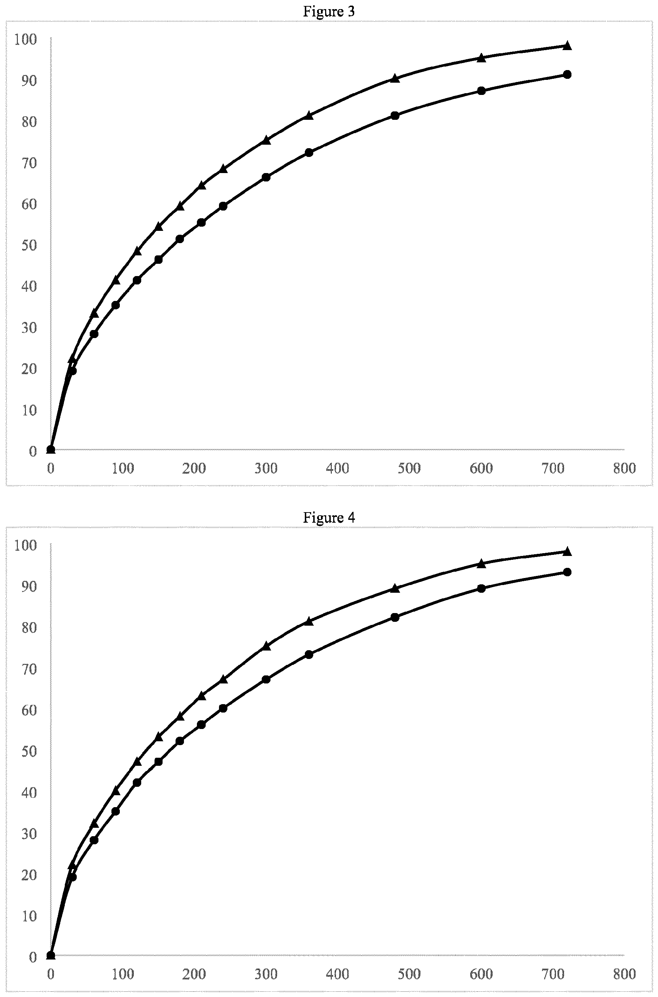

Figure 3 shows *in vitro* release profiles of comparative Example 1-2. The *in vitro* release profile of a conventional intact scored tablet (intact tablet, •) is compared with the *in vitro* release profile of a single half after the conventional scored tablet has been broken apart along its site of mechanical weakness (split tablet, A). As demonstrated, the *in vitro* release profiles significantly differ from one another.

Figure 4 shows *in vitro* release profiles of comparative Example 1-3. The *in vitro* release profile of a conventional intact scored tablet (intact tablet, •) is compared with the *in vitro* release profile of a single half after the conventional scored tablet has been broken apart along its site of mechanical weakness (split tablet, A). As demonstrated, the *in vitro* release profiles significantly differ from one another.

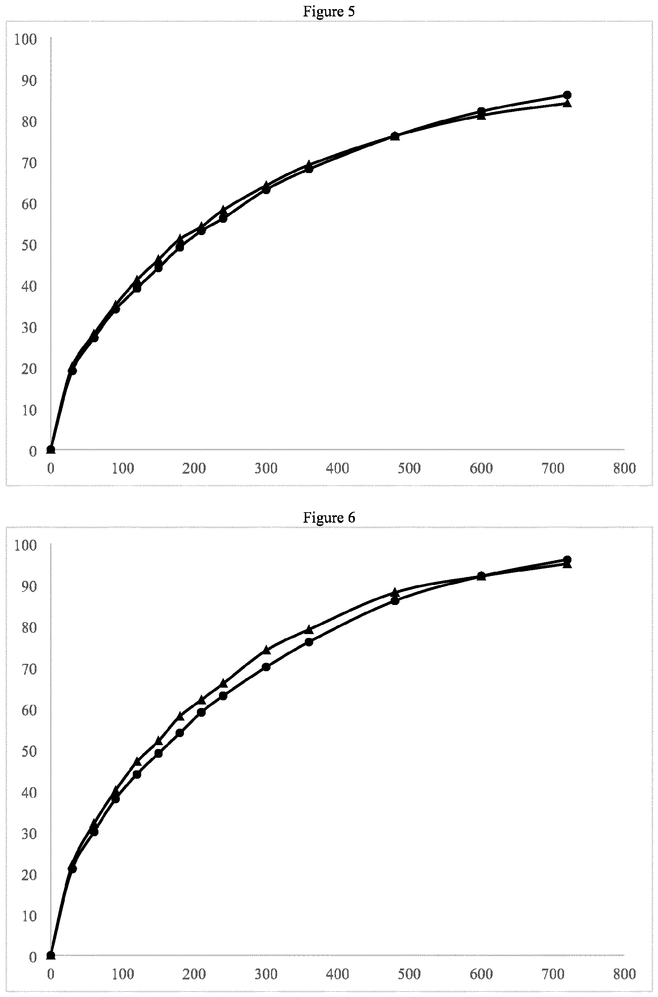

Figure 5 shows *in vitro* release profiles of inventive Example 2. The *in vitro* release profile of an intact scored tablet according to the invention (intact tablet, •) is compared with the *in vitro* release profile of a single half after the scored tablet according to the invention has been broken apart along its site of mechanical weakness (split tablet, ▲)**.** As demonstrated, the *in vitro* release profiles do not significantly differ from one another.

Figure 6 shows *in vitro* release profiles of inventive Example 3. The *in vitro* release profile of an intact scored tablet according to the invention (intact tablet, •) is compared with the *in vitro* release profile of a single half after the scored tablet according to the invention has been broken apart along its site of mechanical weakness (split tablet, ▲)**.** As demonstrated, the *in vitro* release profiles do not significantly differ from one another.

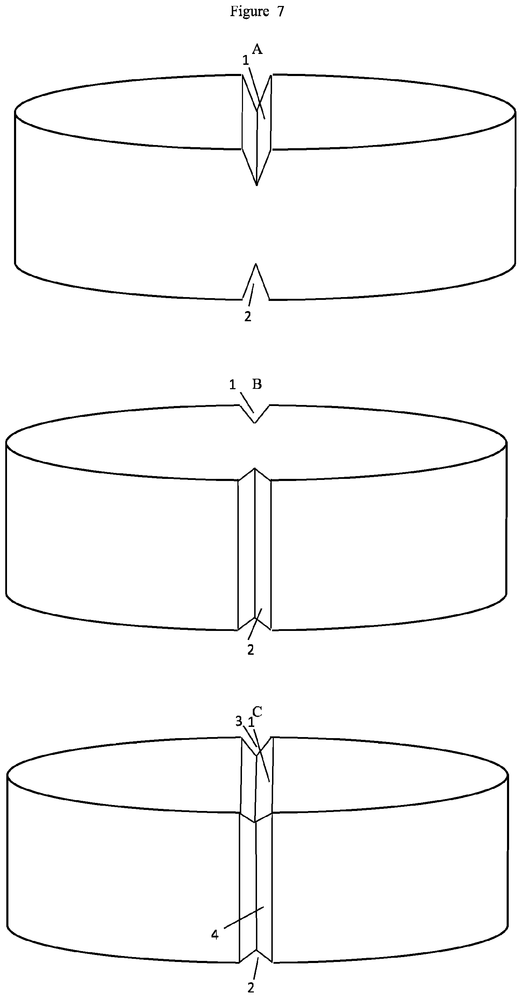

Figure 7 schematically illustrates different embodiments of sites of mechanical weakness. Figure 7A is a perspective view of a preferred embodiment of a scored tablet having a first score (1) and a second score (2) on opposite sides. The scored tablet has cylindrical shape and the first score (1) and the second score (2) are placed within the circular faces of the cylinder. Figure 7B is a perspective view of another embodiment of a scored tablet having a first score (1) and a second score (2) on opposite sides. The scored tablet has cylindrical shape and the first score (1) and the second score (2) are placed within the cylinder barrel. Figure 7C is a perspective view of another embodiment of a scored tablet having a first score (1), a second score (2), a third score (3), and a fourth score (4), where the first score (1) and the second score (2) are located on opposite sides like in the embodiment shown in Figure 4A, and where the third score (3) and the fourth score (4) are on opposite sides like in the embodiment shown in Figure 4B. The scored tablet has cylindrical shape and the first score (1), the second score (2), the third score (3) and the fourth score (4) together form a circumferential score about the longitudinal axis (5) of the scored tablet.

The scored tablet according to the invention has a site of mechanical weakness along which it can be manually broken into two separate halves preferably having essentially the same size, shape and weight. In other words, the scored tablet according to the invention is a divisible tablet. The score (notch) or scores (notches) can be used to facilitate the splitting of the tablet into fractions when less than a full tablet is desired for a dose or when the patient has problems in swallowing the tablet as a whole. The site of mechanical weakness is preferably formed by one or more scores (notches).

In a preferred embodiment, the scored tablet according to the invention has a longitudinal axis, wherein the site of mechanical weakness is formed by a circumferential score about the longitudinal axis.

In a preferred embodiment, the site of mechanical weakness is formed by a first score, a second score, a third score and a fourth score; wherein the first score is opposite to the second score; wherein the third score is opposite to the fourth score; and wherein the first and second score are perpendicular to the third and fourth score.

Preferably, the site of mechanical weakness lies within a symmetrical plane of the scored tablet.

Preferably, the scored tablet according to the invention is oblong.

Preferably, the scored tablet according to the invention is monolithic. For the purpose of the specification, monolithic means that the scored tablet e.g. does not comprise a multitude of particles that are optionally dispersed in and compressed in an outer matrix material. For example, Multiple Unit Particulate Systems (MUPS) containing sustained release pellets compressed into a fast disintegrating matrix are not monolithic.

Preferably, Tapentadol or the physiologically acceptable salt thereof and the prolonged release matrix material are homogeneously distributed over the scored tablet as a uniform mixture. When the scored tablet is film coated, the coated core of the scored tablet is preferably such uniform mixture, i.e. Tapentadol or the physiologically acceptable salt thereof and the prolonged release matrix material are then homogeneously distributed over the core of the scored tablet as a uniform mixture.

The scored tablet according to the invention comprises as pharmacologically active ingredient Tapentadol or a physiologically acceptable salt thereof.

Providing Tapentadol or a physiologically acceptable salt thereof in scored tablets can serve different purposes. On the one hand, it may be desirable to offer to the patient pharmaceutical dosage forms that may be divided into smaller units for ease of swallowing. On the other hand, it may be desirable to initiate pharmacological treatment at a comparatively low dose of Tapentadol or the physiologically acceptable salt thereof and to consecutively increase the dose of Tapentadol or the physiologically acceptable salt thereof over time, i.e. to titrate the administered dose, until the therapeutically effective dose is reached providing optimized therapeutic benefits at minimized treatment emergent adverse events.

Preferably, at the initial phase of pharmacological treatment titration of Tapentadol or the physiologically acceptable salt thereof is intended.

Preferably, the weight content of Tapentadol or the physiologically acceptable salt thereof ingredient is at least 4.0 wt.-%, more preferably at least 5.0 wt.-%, still more preferably at least 6.0 wt.-%, yet more preferably at least 7.0 wt.-%, even more preferably at least 8.0 wt.-%, most preferably at least 9.0 wt.-%, and in particular at least 10 wt.-%, in each case relative to the total weight of the scored tablet.

Preferably, the weight content of Tapentadol or the physiologically acceptable salt thereof is at most 55 wt.-%, more preferably at most 50 wt.-%, still more preferably at most 47.5 wt.-%, yet more preferably at most 45 wt.-%, even more preferably at most 42.5 wt.-%, most preferably at most 41 wt.-%, and in particular at most 10 wt.-%, in each case relative to the total weight of the scored tablet.

In preferred embodiments, the weight content of Tapentadol or the physiologically acceptable salt thereof is within the range of from 10 to 50 wt.-%, more preferably 20±10 wt.-%, or 25±15 wt.-%, or 25±10 wt.-%, or 30±20 wt.-%, or 30±15 wt.-%, or 30±10 wt.-%, or 35±15 wt.-%, or 35±10 wt.-%, in each case relative to the total weight of the scored tablet.

Preferably, the total dose of Tapentadol or the physiologically acceptable salt thereof is at least 10 mg, more preferably at least 15 mg, still more preferably at least 20 mg, yet more preferably at least 25 mg, even more preferably at least 30 mg, most preferably at least 35 mg, and in particular at least 40 mg, in each case as equivalent weight relative to the non-salt form of Tapentadol.

Preferably, the total dose of Tapentadol or the physiologically acceptable salt thereof is at most 420 mg, more preferably at most 400 mg, still more preferably at most 380 mg, yet more preferably at most 360 mg, even more preferably at most 340 mg, most preferably at most 320 mg, and in particular at most 300 mg, in each case as equivalent weight relative to the non-salt form of Tapentadol.

In preferred embodiments, the total dose of Tapentadol or the physiologically acceptable salt thereof is within the range of from 5.0 to 50 mg, more preferably 25±22.5 mg, still more preferably 25±20 mg, yet more preferably 25±17.5 mg, even more preferably 25±15 mg, most preferably 25±12.5 mg, and in particular 25±10 mg, in each case as equivalent weight relative to the non-salt form of Tapentadol.

In other preferred embodiments, the total dose of Tapentadol or the physiologically acceptable salt thereof is within the range of from 50 to 150 mg, more preferably 50±45 mg or 100±45 mg or 150±45 mg, still more preferably 50±40 mg or 100±40 mg or 150±40 mg, yet more preferably 50±35 mg or 100±35 mg or 150±35 mg, even more preferably 50±20 mg or 100±20 mg or 150±20 mg, most preferably 50±25 mg or 100±25 mg or 150±25 mg, and in particular 50±20 mg or 100±20 mg or 150±20 mg, in each case as equivalent weight relative to the non-salt form of Tapentadol.

In still other preferred embodiments, the total dose of Tapentadol or the physiologically acceptable salt thereof is within the range of from 150 to 300 mg, more preferably 200±45 mg or 250±45 mg, still more preferably 200±40 mg or 250±40 mg, yet more preferably 200±35 mg or 250±35 mg, even more preferably 200±20 mg or 250±20 mg, most preferably 200±25 mg or 250±25 mg, and in particular 200±20 mg or 250±20 mg, in each case as equivalent weight relative to the non-salt form of Tapentadol.

While it is contemplated that the scored tablet according to the invention may contain a combination of two or more pharmacologically active ingredients, the scored tablet preferably contains Tapentadol or the physiologically acceptable salt thereof as single (i.e. the sole) pharmacologically active ingredient.

The scored tablet according to the invention contains a prolonged release matrix material in which Tapentadol or the physiologically acceptable salt thereof is embedded. Preferably, Tapentadol or the physiologically acceptable salt thereof is homogeneously distributed within the prolonged release matrix material.

The prolonged release matrix material contains at least one physiologically acceptable polymer that serves the purpose of retarding release of Tapentadol or the physiologically acceptable salt thereof from the scored tablet. The prolonged release matrix material additionally contains a binder and preferably a lubricant. The prolonged release matrix material may comprise further conventional excipients such as antioxidants.

Preferably, the prolonged release matrix material is composed, i.e. essentially consists of at least one physiologically acceptable polymer that serves the purpose of retarding release of Tapentadol or the physiologically acceptable salt thereof from the scored tablet, at least one binder and at least one lubricant. For the purpose of the specification, unless expressly stated otherwise, the prolonged release matrix material is composed of all excipients that are contained in the scored tablet, i.e. all ingredients except Tapentadol or the physiologically acceptable salt thereof, and when the scored tablet is film coated, except the film coating material.

The weight content of the prolonged release matrix material is at least 45 wt.-%, yet more preferably at least 47.5 wt.-%, even more preferably at least 50 wt.-%, most preferably at least 52.5 wt.-%, and in particular at least 55 wt.-%, in each case relative to the total weight of the scored tablet.

Preferably, the weight content of the prolonged release matrix material is at most 94 wt.-%, more preferably at most 93 wt.-%, still more preferably at most 92 wt.-%, yet more preferably at most 91 wt.-%, even more preferably at most 90 wt.-%, most preferably at most 89 wt.-%, and in particular at most 88 wt.-%, in each case relative to the total weight of the scored tablet.

Preferably, the weight content of the prolonged release matrix material is within the range of from 55 to 90 wt.-%; more preferably 60±5 wt.-%, or 65±10 wt.-%, or 65±5 wt.-%, or 70±15 wt.-%, or 70±10 wt.-%, or 70±5 wt.-%, or 75±15 wt.-%, or 75±10 wt.-%, or 75±5 wt.-%, or 80±10 wt.-%, or 80±5 wt.-%, or 85±5 wt.-%, in each case relative to the total weight of the scored tablet.

The prolonged release matrix material contains hydroxypropylmethylcellulose as at least one physiologically acceptable polymer that serves the purpose of retarding release of Tapentadol or the physiologically acceptable salt thereof from the scored tablet and that also has a comparatively high water solubility. In other words, Tapentadol or the physiologically acceptable salt thereof and the at least one physiologically acceptable polymer are both hydrophile.

According to USP, solubilities of substances are quantified in terms of parts of solvent required for 1 part of solute according to the following scale: very soluble = less than 1, freely soluble = from 1 to 10, soluble = from 10 to 30, sparingly soluble = from 30 to 100, slightly soluble = from 100 to 1000, very slightly soluble = from 1000 to 10,000, and practically insoluble, or insoluble = 10,000 and over.

Preferably, in accordance with this solubility scale in pure water at 23°C, Tapentadol or the physiologically acceptable salt thereof in its form as contained in the scored tablet according to the invention, i.e. in the non-salt form or the given salt form, is at least sparingly soluble (less than 100 parts of solvent required for 1 part of solute), more preferably at least soluble (less than 30 parts of solvent required for 1 part of solute), still more preferably at least freely soluble (less than 10 parts of solvent required for 1 part of solute), and most preferably very soluble (less than 1 part of solvent required for 1 part of solute).

Preferably, the at least one physiologically acceptable polymer that serves the purpose of retarding release of Tapentadol or the physiologically acceptable salt thereof from the scored tablet dissolves in cold water by swelling and subsequent hydration. As far as such hydrophilic polymers are concerned, there is typically no sharp solubility limit such as occurs in the dissolution of ionizing salts. The concentration of such hydrophilic polymers in solution is usually limited by the viscosity that a manufacturer is equipped to handle. It also depends on the viscosity and chemical type of hydrophilic polymer used. Solutions of low-viscosity polymers can be made at 10% to 15% concentration while high-viscosity polymers have a normal limit at 2% to 3% concentration.

Preferably, in accordance with the above solubility scale in pure water at 23°C, the at least one physiologically acceptable polymer is at least slightly soluble (less than 1000 parts of solvent required for 1 part of solute), more preferably at least sparingly soluble (less than 100 parts of solvent required for 1 part of solute), still more preferably at least soluble (less than 30 parts of solvent required for 1 part of solute).

The scored tablet according to the invention, the prolonged release matrix material, contains at least one physiologically acceptable polymer that serves the purpose of retarding release of Tapentadol or the physiologically acceptable salt thereof from the scored tablet. Thus, said at least one physiologically acceptable polymer is part of the prolonged release material of the scored tablet according to the invention.

The prolonged release matrix material comprises a cellulose derivative being hydroxypropylmethyl cellulose.

The cellulose derivative is hydroxypropylmethylcellulose. Preferably, the hydroxypropylmethylcellulose is selected from hypromellose type 1828, 2208, 2906 and 2910 according to USP having the following methoxyl content hydroxypropoxyl content:

| | methoxyl (%) | | hydroxypropoxyl (%) | |
|---|---|---|---|---|
| type | min | max | min | max |
| 1828 | 16.5 | 20.0 | 23.0 | 32.0 |
| 2208 | 19.0 | 24.0 | 4.0 | 12.0 |
| 2906 | 27.0 | 30.0 | 4.0 | 7.5 |
| 2910 | 28.0 | 30.0 | 7.0 | 12.0 |

Preferably, the number average molecular weight Mₙ of the physiologically acceptable polymer, preferably cellulose derivative, more preferably hydroxypropylmethylcellulose, is not more than 220,000 g/mol, more preferably not more than 180,000 g/mol, still more preferably not more than 140,000 g/mol, yet more preferably not more than 120,000 g/mol, even more preferably not more than 110,000 g/mol, most preferably not more than 86,000 g/mol, and in particular not more than 63,000 g/mol.

Preferably, the labelled viscosity of the physiologically acceptable polymer, preferably cellulose derivative, more preferably hydroxypropylmethylcellulose, in accordance with USP (preferably rotor no. 4, revolution 3 rpm, calculation multiplier 2000) is within the range of 100,000±50,000 mPa·s, more preferably 100,000±40,000 mPa·s, still more preferably 100,000±30,000 mPa·s, 100,000±20,000 mPa·s, and most preferably 100,000±10,000 mPa·s.

The weight content of the physiologically acceptable polymer, hydroxypropylmethylcellulose, is at least 10 wt.-%, even more preferably at least 11 wt.-%, most preferably at least 12 wt.-%, and in particular at least 13 wt.-%, in each case relative to the total weight of the scored tablet.

The weight content of the physiologically acceptable polymer, hydroxypropylmethylcellulose, is at most 50 wt.-%, and in particular at most 47.5 wt.-%, in each case relative to the total weight of the scored tablet.

The weight content of the physiologically acceptable polymer, hydroxypropylmethylcellulose, is within the range of from 10 to 50 wt.-%; more preferably 15±5 wt.-%, or 20±10 wt.-%, or 20±5 wt.-%, or 25±15 wt.-%, or 25±10 wt.-%, or 25±5 wt.-%, or 30±20 wt.-%, or 30±15 wt.-%, or 30±10 wt.-%, or 30±5 wt.-%, or 35±15 wt.-%, or 35±10 wt.-%, or 35±5 wt.-%, or 40±10 wt.-%, or 40±5 wt.-%, or 45±5 wt.-%, in each case relative to the total weight of the scored tablet.

The scored tablet according to the invention, the prolonged release matrix material comprises a binder being microcrystalline cellulose within the range of from 35 to 70 wt.-%, relative to the total weight of the scored tablet.

Preferably, a further binder is selected from the group consisting of cellulose, magnesium-aluminum silicates (e.g. bentonite), mono-, oligo- and polysaccharides (e.g. dextrose, lactose, mannose), sugar alcohols (e.g. lactitol, mannitol), starches (e.g. pregelatinized starch, hydrolyzed starch, modified starch), calcium phosphate, polyvinylpyrrolidone, and vinylpyrrolidone/vinyl acetate copolymers; more preferably silicified microcrystalline cellulose.

The weight content of the binder is at least 35 wt.-%, relative to the total weight of the scored tablet.

The weight content of the binder is at most 70 wt.-%, relative to the total weight of the scored tablet.

The weight content of the binder is within the range of from 35 to 70 wt.-%; more preferably 40±5 wt.-%, or 45±10 wt.-%, or 45±5 wt.-%, or 50±15 wt.-%, or 50±10 wt.-%, or 50±5 wt.-%, or 55±15 wt.-%, or 55±10 wt.-%, or 55±5 wt.-%, or 60±10 wt.-%, or 60±5 wt.-%, or 65±5 wt.-%, in each case relative to the total weight of the scored tablet.

When the scored tablet contains more than one binder, the above percentages refer to the total content of all binders that are contained in the scored tablet.

Preferably, the scored tablet according to the invention comprises a lubricant.

Preferably, the lubricant is selected from the group consisting of salts of fatty acids (e.g. magnesium stearate, calcium stearate, zinc stearate), fatty acids (e.g. stearic acid, palmitic acid), glyceryl fatty acid esters (e.g. glyceryl monostearate, glyceryl monobehenate, glyceryl dibehenate, glyceryl tribehenate), sorbitan monostearate, sucrose monopalmitate, sodium stearyl fumarate, hydrated magnesium silicate, and talc; preferably magnesium stearate.

Preferably, the weight content of the lubricant is at least 0.20 wt.-%, more preferably at least 0.25 wt.-%, still more preferably at least 0.30 wt.-%, yet more preferably at least 0.35 wt.-%, even more preferably at least 0.40 wt.-%, most preferably at least 0.45 wt.-%, and in particular at least 0.50 wt.-%, in each case relative to the total weight of the scored tablet.

Preferably, the weight content of the lubricant is at most 2.0 wt.-%, more preferably at most 1.5 wt.-%, still more preferably at most 1.0 wt.-%, yet more preferably at most 0.90 wt.-%, even more preferably at most 0.80 wt.-%, most preferably at most 0.70 wt.-%, and in particular at most 0.60 wt.-%, in each case relative to the total weight of the scored tablet.

Preferably, the weight content of the lubricant is within the range of from 0.1 to 1.0 wt.-%, more preferably 0.50±0.45 wt.-%, still more preferably 0.50±0.40 wt.-%, yet more preferably 0.50±0.35 wt.-%, even more preferably 0.50±0.30 wt.-%, most preferably 0.50±0.25 wt.-%, and in particular 0.50±0.20 wt.-%, in each case relative to the total weight of the scored tablet.

Preferred embodiments A¹ to A¹² of the scored tablet according to the invention, where in each case
- the prolonged release matrix material comprises a binder selected from microcrystalline cellulose; and
- the prolonged release matrix material comprises a cellulose ether selected from hydroxypropylmethylcellulose;
are compiled in the table here below:

| | A¹ | A² | A³ |
|---|---|---|---|
| total weight [mg] | 540±80 | 540±60 | 540±40 |
| Tapentadol [wt.-%]^{1,2} | 22±8 | 22±6 | 22±4 |
| binder [wt.-%]² | 57±8 | 57±6 | 57±4 |
| cellulose ether [wt.-%]² | 19±8 | 19±6 | 19±4 |

| | A⁴ | A⁵ | A⁶ |
|---|---|---|---|
| total weight [mg] | 540±80 | 540±60 | 540±40 |
| Tapentadol [wt.-%]^{1,2} | 32±8 | 32±6 | 32±4 |
| binder [wt.-%]² | 46±8 | 46±6 | 46±4 |
| cellulose ether [wt.-%]² | 19±8 | 19±6 | 19±4 |

| | A⁷ | A⁸ | A⁹ |
|---|---|---|---|
| total weight [mg] | 720±80 | 720±60 | 720±40 |
| Tapentadol [wt.-%]^{1,2} | 33±8 | 33±6 | 33±4 |
| binder [wt.-%]² | 51±8 | 51±6 | 51±4 |
| cellulose ether [wt.-%]² | 19±8 | 19±6 | 19±4 |

| | A¹⁰ | A¹¹ | A¹² |
|---|---|---|---|
| total weight [mg] | 720±80 | 720±60 | 720±40 |
| Tapentadol [wt.-%]^{1,2} | 41±8 | 41±6 | 41±4 |
| binder [wt.-%]² | 43±8 | 43±6 | 43±4 |
| cellulose ether [wt.-%]² | 19±8 | 19±6 | 19±4 |

| | | | |
|---|---|---|---|
| ¹ based upon the total weight of the salt, e.g. hydrochloride ² relative to the total weight of the scored tablet | | | |

Preferably, the scored tablet according to the invention under *in vitro* conditions according to Ph. Eur. 7.0, 2.9.3, paddle apparatus, 50 rpm at 37±0.5 °C in 900 ml aqueous phosphate buffer at pH 6.8 provides, preferably in the intact state as well as in the split state, a release profile such that
- after 30 minutes 20±10%,
- after 60 minutes 29±10%,
- after 120 minutes 43±10%,
- after 360 minutes 73±10%,
- after 480 minutes 81±10%, and
- after 720 minutes 90±10%
of Tapentadol or the physiologically acceptable salt thereof that was originally contained in the scored tablet have been released.

Preferred *in vitro* release profiles B¹ to B⁸ of the scored tablet according to the invention, preferably in the intact state as well as in the split state, under the above stated release conditions are compiled in the table here below:

| time | B¹ | B² | B³ | B⁴ | B⁵ | B⁶ | B⁷ | B⁸ |
|---|---|---|---|---|---|---|---|---|
| 30 min | 20±9% | 20±8% | 20±7% | 20±6% | 20±5% | 20±4% | 20±3% | 20±2% |
| 60 min | 29±9% | 29±8% | 29±7% | 29±6% | 29±5% | 29±4% | 29±3% | 29±2% |
| 120 min | 43±9% | 43±8% | 43±7% | 43±6% | 43±5% | 43±4% | 43±3% | 43±2% |
| 360 min | 73±9% | 73±8% | 73±7% | 73±6% | 73±5% | 73±4% | 73±3% | 73±2% |
| 480 min | 81±9% | 81±8% | 81±7% | 81±6% | 81±5% | 81±4% | 81±3% | 81±2% |
| 720 min | 90±9% | 90±8% | 90±7% | 90±6% | 90±5% | 90±4% | 90±3% | 90±2% |

Preferably, under *in vitro* conditions according to Ph. Eur. 7.0, 2.9.3, paddle apparatus, 50 rpm at 37±0.5 °C in 900 ml aqueous phosphate buffer at pH 6.8 the release profile of a single half deviates from the release profile of the intact scored tablet at any point in time absolutely (calculated absolute difference of released percentages) by at most 10%, more preferably at most 7.5%, still more preferably at most 5.0%, yet more preferably at most 4.5%, even more preferably at most 4.0%, most preferably at most 3.5%, and in particular at most 3.0%.

Preferably, under *in vitro* conditions according to Ph. Eur. 7.0, 2.9.3, paddle apparatus, 50 rpm at 37±0.5 °C in 900 ml aqueous phosphate buffer at pH 6.8 the release profile of a single half deviates from the release profile of the intact scored tablet at any point in time relatively (change expressed in percent relative to intact tablet) by at most 10%, more preferably at most 9%, still more preferably at most 8%, yet more preferably at most 7%, even more preferably at most 6%, most preferably at most 5%, and in particular at most 4%.

Preferably, under *in vitro* conditions according to Ph. Eur. 7.0, 2.9.3, paddle apparatus, 50 rpm at 37±0.5 °C in 900 ml aqueous phosphate buffer at pH 6.8 the release profile of a single half and the release profile of the intact scored tablet meet the similarity factor (f₂) (f₂ = 50·log{[1+(1/n)Σₜ₌₁ⁿ (Rₜ - Tₜ)²]^{-0.5}·100}), where the f₂ value is preferably at least 72, more preferably at least 74, still more preferably at least 76, yet more preferably at least 78, even more preferably at least 80, most preferably at least 82, and in particular at least 84.

The scored tablet according to the invention has been prepared by dry granulation; preferably by slugging method or roller compactor method.

In slugging also known as precompression the material to be granulized is first made into a large compressed mass or "slug" typically by way of a tablet press using large flat-faced tooling. A fairly dense slug may be formed by allowing sufficient time for the air to escape from the material to be compacted. Compressed slugs are then comminuted through a desired mesh screen manually or automatically as for example by way of a comminuting mill.

In a roller compactor material particles are consolidated and densified by passing the material under pressure between two counter-rotating rolls. The densified material or "ribbon" from a roller compactor is then reduced to a uniform granule size in a granulator by comminuted through a desired mesh screen.

Preferably, the scored tablet according to the invention has a tablet porosity of at most 0.15, more preferably at most 0.14, still more preferably at most 0.13, yet more preferably at most 0.12, even more preferably at most 0.11, most preferably at most 0.10, and in particular at most 0.09.

For the purpose of the specification, tablet porosity ε is calculated using the equation ε = 1-(ρ_{tablet} - ρₜᵣᵤₑ), where ρ_{tablet} is the tablet apparent density and ρₜᵣᵤₑ is the true density of the powder mixture or granule samples measured by multipycnometer (e.g. MVP-D160-E, Quantachrome Instruments, USA; n=5, helium pressure 1013 hPa, difference in helium pressure before and after sample loading is recorded to determine the true volume of the samples).

Preferably, the scored tablet according to the invention has a breaking strength according to Ph. Eur. 2.9.8 of at least 100 N, more preferably at least 110 N, still more preferably at least 120 N, yet more preferably at least 125 N, even more preferably at least 130 N, most preferably at least 135 N, and in particular at least 140 N.

Preferably, the scored tablet according to the invention has a breaking strength according to Ph. Eur. 2.9.8 of at most 260 N, more preferably at most 250 N, still more preferably at most 240 N, yet more preferably at most 230 N, even more preferably at most 220 N, most preferably at most 210 N, and in particular at most 200 N.

In a preferred embodiment, the scored tablet according to the invention has a breaking strength according to Ph. Eur. 2.9.8 within the range of from 100 to 200 N, more preferably 150±45 N, still more preferably 150±40 N, yet more preferably 150±35 N, even more preferably 150±30 N, most preferably 150±25 N, and in particular 150±20 N.

In another preferred embodiment, the scored tablet according to the invention has a breaking strength according to Ph. Eur. 2.9.8 within the range of from 125 to 225 N, more preferably 175±45 N, still more preferably 175±40 N, yet more preferably 175±35 N, even more preferably 175±30 N, most preferably 175±25 N, and in particular 175±20 N.

In still another preferred embodiment, the scored tablet according to the invention has a breaking strength according to Ph. Eur. 2.9.8 within the range of from 150 to 250 N, more preferably 200±45 N, still more preferably 200±40 N, yet more preferably 200±35 N, even more preferably 200±30 N, most preferably 200±25 N, and in particular 200±20 N.

Preferably, the scored tablet according to the invention has a tablet apparent density of at least 1.20 g·cm⁻³, at least 1.21 g·cm⁻³, at least 1.22 g·cm⁻³, at least 1.23 g·cm⁻³, at least 1.24 g·cm⁻³, at least 1.25 g·cm⁻³, at least 1.26 g·cm⁻³, at least 1.27 g·cm⁻³, at least 1.28 g·cm⁻³, or at least 1.29 g·cm⁻³.

Preferably, the scored tablet according to the invention has a tablet apparent density of at most 1.31 g·cm⁻³, at most 1.32 g·cm⁻³, at most 1.33 g·cm⁻³, at most 1.34 g·cm⁻³, at most 1.35 g·cm⁻³, at most 1.36 g·cm⁻³, at most 1.37 g·cm⁻³, at most 1.38 g·cm⁻³, at most 1.39 g·cm⁻³, or at most 1.40 g·cm⁻³.

In preferred embodiments, the scored tablet according to the invention has a tablet apparent density within the range of 1.22±0.03 g·cm⁻³, 1.24±0.03 g·cm⁻³, 1.26±0.03 g·cm⁻³, 1.28±0.03 g·cm⁻³, 1.30±0.03 g·cm⁻³, 1.32±0.03 g·cm⁻³, 1.34±0.03 g·cm⁻³, 1.36±0.03 g·cm⁻³, or 1.38±0.03 g·cm⁻³.

When the scored tablet is film-coated with a film coating material, the contribution of the film coating material (weight and volume) is disregarded with respect to apparent density, i.e. the above values refer to the core of the scored tablet according to the invention.

Preferably, the scored tablet according to the invention has a total weight within the range of from 200 to 750 mg.

In a preferred embodiment, the scored tablet according to the invention has a total weight of at least 80 mg, more preferably at least 100 mg, still more preferably at least 120 mg, yet more preferably at least 140 mg, even more preferably at least 160 mg, most preferably at least 180 mg, and in particular at least 200 mg. Preferably, the scored tablet according to the invention has a total weight of at most 360 mg, more preferably at most 340 mg, still more preferably at most 320 mg, yet more preferably at most 300 mg, even more preferably at most 280 mg, most preferably at most 260 mg, and in particular at most 240 mg. Preferably, the scored tablet according to the invention has a total weight within the range of from 50 mg to 400 mg, more preferably 220±150 mg, still more preferably 220±125 mg, yet more preferably 220±100 mg, even more preferably 220±75 mg, most preferably 220±50 mg, and in particular 220±25 mg. Preferably, according to this embodiment, the dose of Tapentadol or the physiologically acceptable salt thereof that is contained in the scored tablet, preferably Tapentadol hydrochloride, is about 25 mg, relative to the equivalent weight of the free base of Tapentadol (non-salt form).

In another preferred embodiment, the scored tablet according to the invention has a total weight of at least 200 mg, more preferably at least 250 mg, still more preferably at least 300 mg, yet more preferably at least 350 mg, even more preferably at least 400 mg, most preferably at least 450 mg, and in particular at least 500 mg. Preferably, the scored tablet according to the invention has a total weight of at most 850 mg, more preferably at most 800 mg, still more preferably at most 750 mg, yet more preferably at most 700 mg, even more preferably at most 650 mg, most preferably at most 600 mg, and in particular at most 550 mg. Preferably, the scored tablet according to the invention has a total weight within the range of from 300 mg to 700 mg, more preferably 540±150 mg, still more preferably 540±125 mg, yet more preferably 540±100 mg, even more preferably 540±75 mg, most preferably 540±50 mg, and in particular 540±25 mg. Preferably, according to this embodiment, the dose of Tapentadol or the physiologically acceptable salt thereof that is contained in the scored tablet, preferably Tapentadol hydrochloride, is about 50 mg, about 100 mg, or about 150 mg, relative to the equivalent weight of the free base of Tapentadol (non-salt form).

In still another preferred embodiment, the scored tablet according to the invention has a total weight of at least 400 mg, more preferably at least 450 mg, still more preferably at least 500 mg, yet more preferably at least 550 mg, even more preferably at least 600 mg, most preferably at least 650 mg, and in particular at least 700 mg. Preferably, the scored tablet according to the invention has a total weight of at most 1050 mg, more preferably at most 1000 mg, still more preferably at most 950 mg, yet more preferably at most 900 mg, even more preferably at most 850 mg, most preferably at most 800 mg, and in particular at most 750 mg. Preferably, the scored tablet according to the invention has a total weight within the range of from 500 mg to 900 mg, more preferably 720±150 mg, still more preferably 720±125 mg, yet more preferably 720±100 mg, even more preferably 720±75 mg, most preferably 720±50 mg, and in particular 720±25 mg. Preferably, according to this embodiment, the dose of Tapentadol or the physiologically acceptable salt thereof that is contained in the scored tablet, preferably Tapentadol hydrochloride, is about 200 mg or about 250 mg, relative to the equivalent weight of the free base of Tapentadol (non-salt form).

Another aspect of the invention relates to a process for the preparation of a scored tablet according to the invention, the process comprising the steps of
(a) providing a mixture comprising Tapentadol or a physiologically acceptable salt thereof and prolonged release matrix material;
(b) compressing said mixture thereby obtaining a compacted material;
(c) breaking said compacted material thereby obtaining a dry granulate material;
(d) optionally, sieving the dry granulate material;
(e) optionally, adding a lubricant;
(f) compressing the dry granulate material thereby obtaining the scored tablet; and
(g) optionally, coating the scored tablet with a film coat.

Preferably, the mixture provided in step (a) of the process according to the invention additionally comprises a lubricant. This variant is particularly relevant when the process according to the invention is a slugging process.

Preferably, the mixture provided in step (a) of the process according to the invention has a humidity content within the range of from 1.0 to 8.0%, more preferably 2.0 to 7.0%, still more preferably 3.0 to 6.0%.

Preferably, step (b) of the process according to the invention is performed by means of a roller compactor, an eccentric press, or a rotary press; preferably a roller compactor.

In a preferred embodiment, in step (b) of the process according to the invention the mixture is compressed at a pressing force of at least 8 kN, more preferably at least 9 kN, still more preferably at least 10 kN, yet more preferably at least 11 kN, even more preferably at least 12 kN, most preferably at least 13 kN, and in particular at least 14 kN. This variant is particularly relevant when the process according to the invention is a slugging process. Preferably, in step (b) of the process according to the invention the mixture is compressed at a pressing force of at most 23 kN, more preferably at most 22 kN, still more preferably at most 21 kN, yet more preferably at most 20 kN, even more preferably at most 19 kN, most preferably at most 18 kN, and in particular at most 17 kN. This variant is particularly relevant when the process according to the invention is a slugging process.

In another preferred embodiment, in step (b) of the process according to the invention the mixture is compressed at a pressing force of at most 4.2 kN/cm, more preferably at most 4.0 kN/cm, still more preferably at most 3.8 kN/cm, yet more preferably at most 3.6 kN/cm, even more preferably at most 3.4 kN/cm, most preferably at most 3.2 kN/cm, and in particular at most 3.0 kN/cm. This variant is particularly relevant when the process according to the invention is a roller compaction process.

In a preferred embodiment, the compacted material obtained in step (b) of the process according to the invention has a higher breaking strength than the scored tablet obtained in step (f). This variant is particularly relevant when the process according to the invention is a slugging process.

Preferably, the compacted material obtained in step (b) of the process according to the invention has a breaking strength according to Ph. Eur. 2.9.8 of at least 160 N, more preferably at least 170 N, still more preferably at least 180 N, yet more preferably at least 190 N, even more preferably at least 200 N or more than 200 N, most preferably at least 210 N, and in particular at least 220 N.

Preferably, the compacted material obtained in step (b) of the process according to the invention has a breaking strength according to Ph. Eur. 2.9.8 of at most 330 N, more preferably at most 320 N, still more preferably at most 310 N, yet more preferably at most 300 N, even more preferably at most 290 N, most preferably at most 280 N, and in particular at most 270 N.

Preferably, the compacted material obtained in step (b) of the process according to the invention has a breaking strength according to Ph. Eur. 2.9.8 within the range of from 200 to 300 N, more preferably 250±45 N, still more preferably 250±40 N, yet more preferably 250±35 N, even more preferably 250±30 N, most preferably 250±25 N, and in particular 250±20 N.

Preferably, step (c) of the process according to the invention is performed by means of a spiked roller, crushing roller, rasp, oscillating granulator, oscillating sieve, or conus sieve.

Preferably, in step (d) of the process according to the invention, coarser particles are disrupted by means of the sieve so that the dry granulate material has a particle size passing a sieve having a mesh size of 1.68 mm. In preferred embodiments, the dry granulate material has a particle size passing a sieve having a mesh size of 1.41 mm, 1.19 mm, 1.00 mm, 0.80 mm, or 0.84 mm.

Preferably, the separated fines are recycled to step (a) of the process according to the invention.

Preferably, step (e) of the process according to the invention is performed, i.e. a lubricant is added to the dry and optionally sieved granulate material. This variant is particularly relevant when the process according to the invention is a roller compaction process.

Preferably, step (f) of the process according to the invention is performed under a higher pressing force than step (b) of the process according to the invention.

Preferably, step (f) of the process according to the invention is performed under a pressing force of at least 10 kN, more preferably at least 11 kN, still more preferably at least 12 kN, yet more preferably at least 13 kN, even more preferably at least 14 kN, most preferably at least 15 kN, and in particular at least 16 kN.

Preferably, step (f) of the process according to the invention is performed under a pressing force of at most 23 kN, more preferably at most 22 kN, still more preferably at most 21 kN, yet more preferably at most 20 kN, even more preferably at most 19 kN, most preferably at most 18 kN, and in particular at most 17 kN.

Another aspect of the invention relates to a scored tablet according to the invention as described above obtainable by the process according to the invention as described above.

Preferably, the scored tablet according to the invention is for administration twice daily, where the desired therapeutic effect due to Tapentadol or the physiologically acceptable salt thereof is preferably provided for at least 8 hours, more preferably for at least 10 hours, still more preferably for at least 12 hours.

Preferably, the scored tablet according to the invention is for oral administration, preferably peroral administration (i.e. by swallowing).

Preferably, the scored tablet according to the invention is for administration to humans, preferably adults and/or geriatric patients.

Another aspect of the invention relates to the scored tablet according to the invention is for use in medical therapy. Another aspect of the invention relates to the use of Tapentadol or a physiologically acceptable salt thereof for the manufacture of the scored tablet according to the invention for medical therapy. Another aspect of the invention relates to a method of medical therapy comprising the administration of the scored tablet according to the invention to a subject in need thereof.

The scored tablet according to the invention is preferably for use in the treatment of analgesia.

Preferably, medical therapy comprises
- a first administration interval comprising at least one day, wherein during the first administration interval one separate half of a scored tablet is administered once daily, twice daily or thrice daily;
- a second administration interval comprising at least one day directly following said first administration interval, wherein during the second administration interval one whole scored tablet is administered once daily, twice daily or thrice daily;
- optionally, a third administration interval comprising at least one day directly following said second administration interval, wherein during the third administration interval a whole scored tablet as well as a separate half of a scored tablet are administered once daily, twice daily or thrice daily; and
- optionally, a fourth administration interval comprising at least one day directly following said third administration interval, wherein during the fourth administration interval two whole scored tablets are administered once daily, twice daily or thrice daily.

When the scored tablet e.g. contains 100 mg of Tapentadol or the physiologically acceptable salt thereof as equivalent weight relative to the non-salt form of Tapentadol,
- during the first administration interval 50 mg of Tapentadol or the physiologically acceptable salt thereof as equivalent weight relative to the non-salt form of Tapentadol are administered once daily, twice daily or thrice daily (i.e. total daily doses of 50 mg, 100 mg or 150 mg);
- during the second administration interval 100 mg of Tapentadol or the physiologically acceptable salt thereof as equivalent weight relative to the non-salt form of Tapentadol are administered once daily, twice daily or thrice daily (i.e. total daily doses of 100 mg, 200 mg or 300 mg);
- optionally, during the third administration interval 150 mg of Tapentadol or the physiologically acceptable salt thereof as equivalent weight relative to the non-salt form of Tapentadol are administered once daily, twice daily or thrice daily (i.e. total daily doses of 150 mg, 300 mg or 450 mg); and
- optionally, during the fourth administration interval 200 mg of Tapentadol or the physiologically acceptable salt thereof as equivalent weight relative to the non-salt form of Tapentadol are administered once daily, twice daily or thrice daily (i.e. total daily doses of 200 mg, 400 mg or 600 mg).

The following examples further illustrate the invention but are not to be construed as limiting its scope.

### Preparation of powder mixture for subsequent processing

Powder mixtures having the following composition were prepared:

| | | |
|---|---|---|
| Tapentadol HCl | 22.19 wt.-% | 116.48 mg |
| hydroxypropylmethylcellulose 100,000 mPa·s | 19.05 wt.-% | 100.00 mg |
| silicified microcrystalline cellulose | 58.19 wt.-% | 305.52 mg |
| magnesium stearate | 0.57 wt.-% | 3.00 mg |
| ∑ | 100.00 wt.-% | 525.00 mg |

Tapentadol HCl, silicified microcrystalline cellulose (ProsolvHD90) and HPMC were separately weighed (Mettler Toledo balance), sieved (Frewitt sieving machine type MF LAB, mesh size 0.8 mm or handsieve, mesh size 1.0 mm), mixed with one another and subsequently blended for 10 minutes by means of a laboratory mixer (Bohle type LM40 or Erweka BKS20). 10 wt.-% of the thus obtained pre-blend were sieved together with separately weighed magnesium stearate (Frewitt sieving machine type MF LAB, mesh size 0.8 mm or handsieve, mesh size 1.0 mm) and the remaining 90% of pre-blend were added. The mixture containing Tapentadol HCl, silicified microcrystalline cellulose, HPMC and magnesium stearate was subsequently blended for 3 minutes by means of a laboratory mixer (Bohle type LM40 or Erweka KBS20).

From the above powder mixture, tablets having a total target weight of 525 mg were prepared using a punch format as further illustrated in Figure 1 (or similar).

### Example 1 - comparative - compression using different prepressing forces:

Tablets were manufactured from the above powder mixture by direct compression using a rotary tablet press (Fette type 102i) operated at a tableting speed of 6,000 h⁻¹. Compression was performed at different prepressing forces and major pressing forces:
Example 1-1: 2.7 kN and 16.0 kN;
Example 1-2: 5.96 kN and 13.99 kN; and
Example 1-3: 9.31 kN and 15.74 kN.

Prepressing generally serves the purpose of removing air that is entrapped between the crystallites that are subsequently to be compressed.

The breaking strength of the tablets was measured (Sotax HT10) and breaking strengths amounted to:
Example 1-1: 263 N;
Example 1-2: 225 N; and
Example 1-3: 243 N.

### Example 2 - inventive - dry granulation by slugging and subsequent compression:

10 Tablets each obtained by Example 1-2 and 1-3 were ground together in a mortar. The obtained powder was sieved (Frewitt sieving machine type MF LAB, mesh size 0.8 mm) and the thus sieved powder mixture was again compressed using a rotary tablet press (Fette type 102i) operated at a tableting speed of 6,000 h⁻¹. Compression was performed at a prepressing force of 2.7 kN and a major pressing force of 16.79 kN.

The breaking strength of the tablets was measured (Sotax HT10) and the breaking strength amounted to 152 N.

### Example 3 - inventive - dry granulation by roller compaction and subsequent compression:

The above powder mix was roller compacted (Gerteis Minipactor) using a roller speed of 3 rpm, a force of 3 kN/cm and a roller gap of 3 mm. The resulting ribbons were granulated using an oscillating granulator (sieve mesh size 1.5 mm).

Tablets were manufactured from the above roller compaction granules using a rotary tablet press (Fette type 102i) operated at a tableting speed of 40,000 h⁻¹. Compression was performed at a prepressing force of xxx and a major pressing force of 16.2 kN.

The breaking strength of the tablets was measured (Sotax HT10) and breaking strength were amounted to 209 N.

Pressing forces and measured breaking strengths are also compiled in the table here below:

| | 1-1 | 1-2 | 1-3 | 2 | 3 |
|---|---|---|---|---|---|
| prepressing force | 2.70 kN | 5.96 kN | 9.31 kN | 2.7 kN | n.d. |
| major pressing force | 16.0 kN | 13.99 kN | 15.74 kN | 16.79 kN | 16.2 kN |
| breaking strength (n=10) | 263 N | 225 N | 243 N | 152 N | 209 N |

The *in vitro* release profile of the scored tablets was measured and compared to the *in vitro* release profile of a single half after manual. Measurements were performed under identical conditions according to Ph. Eur., paddle apparatus, 50 rpm at 37±0.5 °C in 900 ml aqueous phosphate buffer at pH 6.8. The *in vitro* dissolution data are compiled in the table here below:

| min/% | comparative | | | | | | | | inventive | | | | | | | |
|---|---|---|---|---|---|---|---|---|---|---|---|---|---|---|---|---|
| | Example 1-2 | | | | Example 1-3 | | | | Example 2 | | | | Example 3 | | | |
| time | int. | split | Δ | | int. | split | Δ | | int. | split | Δ | | int. | split | Δ | |
| | | | abs | rel | | | abs | rel | | | abs | rel | | | abs | rel |
| 0 | 0 | 0 | 0 | 0 | 0 | 0 | 0 | 0 | 0 | 0 | 0 | 0 | 0 | 0 | 0 | 0 |
| 30 | 19 | 22 | 3 | 18.1 | 19 | 22 | 3 | 13.2 | 19 | 20 | 1 | 5.9 | 21 | 22 | 1 | 7.0 |
| 60 | 28 | 33 | 5 | 18.7 | 28 | 32 | 4 | 13.6 | 27 | 28 | 2 | 5.7 | 30 | 32 | 2 | 6.4 |
| 90 | 35 | 41 | 6 | 17.5 | 35 | 40 | 5 | 13.6 | 34 | 35 | 2 | 5.1 | 38 | 40 | 2 | 6.4 |
| 120 | 41 | 48 | 7 | 17.1 | 42 | 47 | 5 | 13.2 | 39 | 41 | 2 | 4.9 | 44 | 47 | 3 | 6.2 |
| 150 | 46 | 54 | 8 | 16.7 | 47 | 53 | 6 | 13.2 | 44 | 46 | 2 | 4.3 | 49 | 52 | 3 | 6.2 |
| 180 | 51 | 59 | 8 | 16.1 | 52 | 58 | 7 | 12.8 | 49 | 51 | 2 | 4.0 | 54 | 58 | 3 | 6.0 |
| 210 | 55 | 64 | 9 | 15.7 | 56 | 63 | 7 | 12.5 | 53 | 54 | 2 | 3.5 | 59 | 62 | 3 | 5.7 |
| 240 | 59 | 68 | 9 | 15.2 | 60 | 67 | 7 | 12.2 | 56 | 58 | 2 | 3.2 | 63 | 66 | 3 | 5.2 |
| 300 | 66 | 75 | 9 | 13.8 | 67 | 75 | 8 | 11.2 | 63 | 64 | 2 | 2.5 | 70 | 74 | 3 | 4.7 |
| 360 | 72 | 81 | 9 | 12.8 | 73 | 81 | 8 | 10.3 | 68 | 69 | 1 | 1.7 | 76 | 79 | 3 | 3.8 |
| 480 | 81 | 90 | 9 | 10.8 | 82 | 89 | 7 | 8.6 | 76 | 76 | 0 | 0.2 | 86 | 88 | 2 | 2.3 |
| 600 | 87 | 95 | 8 | 9.3 | 89 | 95 | 6 | 6.9 | 82 | 81 | 1 | -1.1 | 92 | 92 | 1 | 0.7 |
| 720 | 91 | 98 | 7 | 8.0 | 93 | 98 | 5 | 5.6 | 86 | 84 | 2 | -1.9 | 96 | 95 | 1 | -0.7 |

| | | | | | | | | | | | | | | | | |
|---|---|---|---|---|---|---|---|---|---|---|---|---|---|---|---|---|
| int. = intact | | | | | | | | | | | | | | | | |

The *in vitro* release profile for the scored tablet according to comparative Example 1-2 and its single half is additionally shown in Figure 3. The *in vitro* release profile for the scored tablet according to comparative Example 1-3 and its single half is additionally shown in Figure 4. The *in vitro* release profile for the scored tablet according to inventive Example 2 and its single half is additionally shown in Figure 5. The *in vitro* release profile for the scored tablet according to inventive Example 3 and its single half is additionally shown in Figure 6.

As demonstrated by Figures 3 through 6, the scored tablets according to the invention (Examples 3 and 4; Figures 5 and 6) have essentially the same dissolution profile, irrespective of whether the scored tablet is intact or broken apart into two separate halves along the breaking score. At any point in time, relative deviations are at most 5.9% (see after 30 minutes, 19.60% - 18.50% = 1.10% (absolute difference); 1.1·100/19.60 = 5.9% (relative difference)).

In contrast, comparative scored tablets (Examples 1-2 and 1-3; Figures 3 and 4) have different dissolution profiles; breaking the scored tablet apart into two separate halves along the breaking score results in an accelerated release profile; *in vitro* release is relatively accelerated by more than 10% during the first 6 hours of release.

## Claims

1. A scored tablet comprising Tapentadol or a physiologically acceptable salt thereof embedded in a prolonged release matrix material;
wherein the prolonged release matrix material comprises a binder selected from microcrystalline cellulose; wherein the weight content of the binder is within the range of from 35 to 70 wt.-%, relative to the total weight of the scored tablet;
wherein the prolonged release matrix material comprises a cellulose derivative selected from hydroxypropylmethylcellulose; wherein the weight content of the cellulose derivative is within the range of from 10 to 50 wt.-%, relative to the total weight of the scored tablet;
wherein the scored tablet has been prepared by dry granulation;
wherein the scored tablet provides prolonged release of Tapentadol or the physiologically acceptable salt thereof;
wherein the scored tablet has a site of mechanical weakness along which it can be manually broken into two separate halves; and
wherein the *in vitro* release profile of the scored tablet essentially corresponds to the *in vitro* release profile of each of the two separate halves.

2. The scored tablet according to claim 1, wherein Tapentadol or the physiologically acceptable salt thereof is Tapentadol hydrochloride.

3. The scored tablet according to claim 1 or 2, which has a total weight within the range of from 200 to 750 mg.

4. The scored tablet according to any of the preceding claims, wherein the weight content of Tapentadol or the physiologically acceptable salt thereof is within the range of from 10 to 50 wt.-%, relative to the total weight of the scored tablet.

5. The scored tablet according to any of the preceding claims, which contains Tapentadol or the physiologically acceptable salt thereof at a total dose within the range of from 25 to 250 mg, as equivalent weight relative to the non-salt form of Tapentadol.

6. The scored tablet according to any of the preceding claims, wherein the weight content of the prolonged release matrix material is within the range of from 55 to 90 wt.-%, relative to the total weight of the scored tablet.

7. The scored tablet according to any of the preceding claims, which comprises a lubricant.

8. The scored tablet according to any of the preceding claims, which has a longitudinal axis, wherein the site of mechanical weakness is formed by a circumferential score about the longitudinal axis.

9. A process for the preparation of a scored tablet according to any of the preceding claims, the process comprising the steps of
(a) providing a mixture comprising Tapentadol or a physiologically acceptable salt thereof and prolonged release matrix material;
(b) compressing said mixture thereby obtaining a compacted material;
(c) breaking said compacted material thereby obtaining a dry granulate material;
(d) optionally, sieving the dry granulate material;
(e) optionally, adding a lubricant;
(f) compressing the dry granulate material thereby obtaining the scored tablet; and
(g) optionally, coating the scored tablet with a film coat.

10. The process according to claim 9, wherein step (b) is performed by means of a roller compactor, an eccentric press, or a rotary press; preferably a roller compactor.

11. The process according to claim 9 or 10, wherein in step (b) the mixture is compressed at a pressing force of at least 10 kN by means of a rotary press.

12. The process according to any of claims 9 to 11, wherein in step (b) the mixture is compressed at a pressing force of at least at least 3.0 kN/cm by means of a roller compactor.

13. The process according to any of claims 9 to 12, wherein in step (d) coarser particles are disrupted by means of the sieve so that the dry granulate material has a particle size passing a sieve having a mesh size of 1.68 mm.

14. The process according to any of claims 9 to 13, wherein step (f) is performed under a higher pressing force than step (b).

15. The process according to any of claims 9 to 14, wherein step (f) is performed under a pressing force of at least 10 kN.

## Patentansprüche

1. Tablette mit Bruchkerbe, umfassend Tapentadol oder ein physiologisch verträgliches Salz davon, eingebettet in ein Matrixmaterial mit verlängerter Freisetzung;
wobei das Matrixmaterial mit verlängerter Freisetzung ein Bindemittel ausgewählt aus mikrokristalliner Cellulose umfasst; wobei der Gewichtsgehalt des Bindemittels im Bereich von 35 bis 70 Gew.-%, bezogen auf das Gesamtgewicht der Tablette mit Bruchkerbe, liegt;
wobei das Matrixmaterial mit verlängerter Freisetzung ein Cellulosederivat ausgewählt aus Hydroxypropylmethylcellulose umfasst; wobei der Gewichtsgehalt des Cellulosederivats im Bereich von 10 bis 50 Gew.-%, bezogen auf das Gesamtgewicht der Tablette mit Bruchkerbe, liegt;
wobei die Tablette mit Bruchkerbe durch Trockengranulierung hergestellt worden ist;
wobei die Tablette mit Bruchkerbe eine verlängerte Freisetzung von Tapentadol oder des physiologisch akzeptablen Salzes davon bereitstellt;
wobei die Tablette mit Bruchkerbe eine Stelle mechanischer Schwäche aufweist, entlang derer sie manuell in zwei separate Hälften geteilt werden kann; und
wobei das In-vitro-Freisetzungsprofil der Tablette mit Bruchkerbe im Wesentlichen dem In-vitro-Freisetzungsprofil jeder der beiden separaten Hälften entspricht.

2. Tablette mit Bruchkerbe nach Anspruch 1, wobei es sich bei Tapentadol oder dem physiologisch verträglichen Salz davon um Tapentadolhydrochlorid handelt.

3. Tablette mit Bruchkerbe nach Anspruch 1 oder 2, die ein Gesamtgewicht im Bereich von 200 bis 750 mg aufweist.

4. Tablette mit Bruchkerbe nach einem der vorhergehenden Ansprüche, wobei der Gewichtsgehalt von Tapentadol oder dem physiologisch akzeptablen Salz davon im Bereich von 10 bis 50 Gew.-%, bezogen auf das Gesamtgewicht der Bruchkerbe, liegt.

5. Tablette mit Bruchkerbe nach einem der vorhergehenden Ansprüche, die Tapentadol oder das physiologisch verträgliche Salz davon in einer Gesamtdosis im Bereich von 25 bis 250 mg, als Äquivalentgewicht bezogen auf die Nichtsalzform von Tapentadol, enthält.

6. Tablette mit Bruchkerbe nach einem der vorhergehenden Ansprüche, wobei der Gewichtsanteil des Matrixmaterials mit verlängerter Freisetzung im Bereich von 55 bis 90 Gew.-%, bezogen auf das Gesamtgewicht der Tablette mit Bruchkerbe, liegt.

7. Tablette mit Bruchkerbe nach einem der vorhergehenden Ansprüche, die ein Schmiermittel umfasst.

8. Tablette mit Bruchkerbe nach einem der vorhergehenden Ansprüche, die eine Längsachse aufweist, wobei die Stelle mechanischer Schwäche durch eine Bruchkerbe umlaufend zur Längsachse gebildet ist.

9. Verfahren zur Herstellung einer Tablette mit Bruchkerbe nach einem der vorhergehenden Ansprüche, wobei das Verfahren die folgenden Schritte umfasst
(a) Bereitstellen einer Mischung, umfassend Tapentadol oder ein physiologisch verträgliches Salz davon und Matrixmaterial mit verlängerter Freisetzung;
(b) Komprimieren der Mischung, wodurch ein kompaktiertes Material erlangt wird;
(c) Auseinanderbrechen des kompaktierten Materials, wodurch ein trockenes Granulatmaterial erlangt wird;
(d) gegebenenfalls Sieben des trockenen Granulatmaterials;
(e) gegebenenfalls Hinzugeben eines Schmiermittels;
(f) Komprimieren des trockenen Granulatmaterials, wodurch eine Tablette mit Bruchkerbe erlangt wird; und
(g) gegebenenfalls Beschichten der Tablette mit Bruchkerbe mit einem Filmüberzug.

10. Verfahren nach Anspruch 9, **dadurch gekennzeichnet, dass** der Schritt (b) durch einen Walzenkompaktor, eine Exzenterpresse oder eine Rotationspresse, bevorzugt durch einen Walzenkompaktor, durchgeführt wird.

11. Verfahren nach Anspruch 9 oder 10, **dadurch gekennzeichnet, dass** in Schritt (b) die Mischung mit einer Presskraft von mindestens 10 kN durch eine Rotationspresse kompaktiert wird.

12. Verfahren nach einem der Ansprüche 9 bis 11, wobei in Schritt (b) die Mischung mit einer Presskraft von mindestens 3,0 kN/cm durch einen Walzenkompaktor kompaktiert wird.

13. Verfahren nach einem der Ansprüche 9 bis 12, wobei in Schritt (d) gröbere Partikel durch das Sieb aufgetrennt werden, sodass das trockene Granulatmaterial eine Partikelgröße aufweist, die ein Sieb mit einer Maschenweite von 1,68 mm passiert.

14. Verfahren nach einem der Ansprüche 9 bis 13, wobei Schritt (f) unter Anwendung einer höheren Presskraft durchgeführt wird als Schritt (b).

15. Verfahren nach einem der Ansprüche 9 bis 14, wobei Schritt (f) unter Anwendung einer Presskraft von mindestens 10 kN durchgeführt wird.

## Revendications

1. Comprimé sécable comprenant du Tapentadol ou un sel physiologiquement acceptable de celui-ci incorporé dans un matériau matriciel à libération prolongée ;
dans lequel le matériau matriciel à libération prolongée comprend un liant choisi dans la cellulose microcristalline ; dans lequel la teneur en poids du liant est comprise dans la plage de 35 à 70 % en poids, par rapport au poids total du comprimé sécable ;
dans lequel le matériau matriciel à libération prolongée comprend un dérivé de cellulose choisi dans l'hydroxypropylméthylcellulose ; dans lequel la teneur en poids du dérivé de cellulose est comprise dans la plage de 10 à 50 % en poids, par rapport au poids total du comprimé sécable ;
dans lequel le comprimé sécable a été préparé par granulation à sec ;
dans lequel le comprimé sécable fournit une libération prolongée de Tapentadol ou du sel physiologiquement acceptable de celui-ci ;
dans lequel le comprimé sécable présente un site de faiblesse mécanique le long duquel il peut être brisé manuellement en deux moitiés séparées ; et
dans lequel le profil de libération *in vitro* du comprimé sécable correspond essentiellement au profil de libération *in vitro* de chacune des deux moitiés séparées.

2. Comprimé sécable selon la revendication 1, dans lequel le Tapentadol ou le sel physiologiquement acceptable de celui-ci est du chlorhydrate de Tapentadol.

3. Comprimé sécable selon la revendication 1 ou 2, qui présente un poids total compris dans la plage de 200 à 750 mg.

4. Comprimé sécable selon l'une quelconque des revendications précédentes, dans lequel la teneur en poids de Tapentadol ou du sel physiologiquement acceptable de celui-ci est comprise dans la plage de 10 à 50 % en poids, par rapport au poids total du comprimé sécable.

5. Comprimé sécable selon l'une quelconque des revendications précédentes, qui contient du Tapentadol ou le sel physiologiquement acceptable de celui-ci à une dose totale comprise dans la plage de 25 à 250 mg, en poids équivalent par rapport à la forme non salée du Tapentadol.

6. Comprimé sécable selon l'une quelconque des revendications précédentes, dans lequel la teneur en poids du matériau matriciel à libération prolongée est comprise dans la plage de 55 à 90 % en poids, par rapport au poids total du comprimé sécable.

7. Comprimé sécable selon l'une quelconque des revendications précédentes, qui comprend un lubrifiant.

8. Comprimé sécable selon l'une quelconque des revendications précédentes, qui présente un axe longitudinal, dans lequel le site de faiblesse mécanique est formé par une strie circonférentielle autour de l'axe longitudinal.

9. Procédé de préparation d'un comprimé sécable selon l'une quelconque des revendications précédentes, le procédé comprenant les étapes consistant à
(a) fournir un mélange comprenant du Tapentadol ou un sel physiologiquement acceptable de celui-ci et un matériau matriciel à libération prolongée ;
(b) comprimer ledit mélange, pour obtenir ainsi un matériau compacté ;
(c) briser ledit matériau compacté, pour obtenir ainsi un matériau granulaire sec ;
(d) éventuellement, tamiser le matériau granulaire sec ;
(e) éventuellement, ajouter un lubrifiant ;
(f) comprimer le matériau granulaire sec pour obtenir ainsi le comprimé sécable ; et
(g) éventuellement, enrober le comprimé sécable avec un pelliculage.

10. Procédé selon la revendication 9, dans lequel l'étape (b) est réalisée au moyen d'un compacteur à rouleaux, d'une presse excentrique ou d'une presse rotative ; de préférence un compacteur à rouleaux.

11. Procédé selon la revendication 9 ou 10, dans lequel, à l'étape (b), le mélange est comprimé à une force de pression d'au moins 10 kN au moyen d'une presse rotative.

12. Procédé selon l'une quelconque des revendications 9 à 11, dans lequel, à l'étape (b), le mélange est comprimé à une force de pression d'au moins 3,0 kN/cm au moyen d'un compacteur à rouleaux.

13. Procédé selon l'une quelconque des revendications 9 à 12, dans lequel, à l'étape (d), les particules plus grossières sont brisées au moyen du tamis de sorte que le matériau granulaire sec présente une taille de particule passant à travers un tamis ayant une taille de mailles de 1,68 mm.

14. Procédé selon l'une quelconque des revendications 9 à 13, dans lequel l'étape (f) est réalisée sous une force de pression plus élevée que l'étape (b).

15. Procédé selon l'une quelconque des revendications 9 à 14, dans lequel l'étape (f) est réalisée sous une force de pression d'au moins 10 kN.
